# EUROPÄISCHE PATENTSCHRIFT

(11) **EP 1 478 382 B1**
(45) Veröffentlichungstag und Bekanntmachung des Hinweises auf die Patenterteilung: **13.12.2006**
(21) Anmeldenummer: 03706506.7
(22) Anmeldetag: 14.02.2003
(51) Int. Cl.: A61K 38/05, A61K 9/12, A61K 31/517

(54) **VERFAHREN ZUR HERSTELLUNG EINES PULVERINHALATIVUMS ENTHALTEND EIN SALZ DES CGRP-ANTAGONISTEN BIBN4096**
METHOD FOR PRODUCING A POWDER INHALANT CONTAINING A SALT OF THE CGRP ANTAGONIST BIBN4096
PROCEDE DE PRODUCTION D'UN INHALANT EN POUDRE CONTENANT UN SEL DE L'ANTAGONISTE DU CGRP BIBN4096

(30) Priorität: 19.02.2002 DE 10206770
(43) Veröffentlichungstag der Anmeldung: 24.11.2004
(73) Patentinhaber: Boehringer Ingelheim Pharma GmbH & Co. KG, 55216 Ingelheim am Rhein (DE)
(72) Erfinder: TRUNK, Michael, 55218 Ingelheim am Rhein (DE); WEILER, Claudius, 55218 Ingelheim-Grosswinterheim (DE)
(86) Internationale Anmeldenummer: PCT/EP2003/001482
(87) Internationale Veröffentlichungsnummer: WO 2003/070753

(56) Entgegenhaltungen:
- WO-A-00/24362
- WO-A-01/10425
- WO-A-01/32144
- WO-A-98/11128
- US-A- 6 165 506
- DOODS H ET AL: "Pharmacological profile of BIBN4096BS, the first selective small molecule CGRP antagonist" BRITISH JOURNAL OF PHARMACOLOGY, BASINGSTOKE, HANTS, GB, Bd. 129, Nr. 3, 2000, Seiten 420-423, XP000992559 ISSN: 0007-1188

## Beschreibung

Die Erfindung betrifft Salze der Substanz 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin [BIBN4096] der Formel I, die unter Normalbedingungen (T < 50°C, relative Feuchte < 75%) in ihrem amorphen Zustand stabil vorliegen, Verfahren zu deren Herstellung, die Verwendung dieser Salze zur Herstellung eines Arzneimittels der Applikationsform Pulverinhalativum, insbesondere zur Herstellung eines Arzneimittels mit CGRPantagonistischen Eigenschaften, sowie ein Verfahren, mit Hilfe dessen in einem einzigen Schritt die thermodynamisch stabilen bzw. stabilisierten Salze des Wirkstoffs im amorphen Zustand zu Mikropartikeln verarbeitet werden können. Nach dem erfindungsgemäßen Verfahren fallen die Salze in Form von sphärisch nanostrukturierten Mikropartikeln an, die zur Herstellung von Pulverinhalativa geeignet sind, bei denen keine weiteren Hilfsstoffe oder Zuschlagsstoffe (Trägermaterialien) benötigt werden, um ein technisch handhabbares Pulver, das sich direkt weiterverarbeiten lässt und das ausgezeichnete Eigenschaften hinsichtlich der Dispergierbarkeit aufweist und bezüglich seiner kohäsiven Eigenschaften ausreichend gut verarbeitbar ist, zu erhalten. Ein weiterer Aspekt der Erfindung sind die mittels des erfindungsgemäßen Verfahrens erhältlichen Pulverinhalativa.

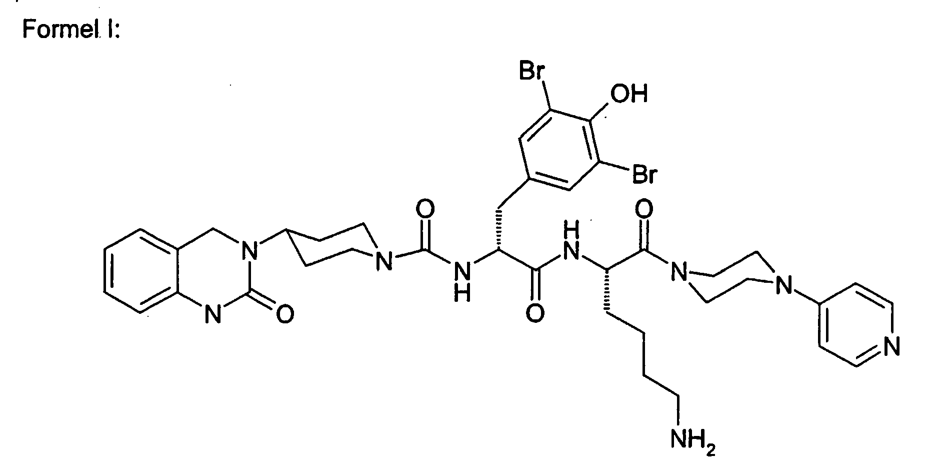

### Stand der Technik

BIBN4096 stellt einen hochwirksamen CGRP-Antagonisten zur Behandlung von Migräne dar, dessen Applikation mittels klassischer Darreichungsformen nicht auf oralem Wege möglich ist, da die Substanz sowohl als Wirkstoffbase als auch in Form ihrer Salze oral nur eine geringe Bioverfügbarkeit aufweist.

Bei der Applikationsform Pulverinhalativa werden Inhalationspulver, die in geeignete Kapseln (Inhaletten) abgefüllt werden, mittels Pulverinhalatoren in die Lunge ausgebracht. Alternativ dazu kann eine inhalative Anwendung auch durch Applikation geeigneter pulverförmiger Inhalationsaerosole, die beispielsweise ein HFA134a, HFA227 oder deren Gemisch als Treibgas enthalten, erfolgen.
Dabei werden die Mikropartikel des reinen Wirkstoffes durch die Atemwege auf der Lungenoberfläche z.B. in den Alveolen mittels des Inhalationsvorganges appliziert. Diese Partikel sedimentieren auf der Oberfläche und können erst nach dem Lösevorgang durch aktive und passive Transportvorgänge im Körper aufgenommen werden.

Die WO 98/11128 offenbart Säureadditionssalze des CGRP-Antagonisten BIBN4096, die in mikronisierter Form und mit einer Oberfläche aus Lezithin in Treibgas-Dosieraerosolen zur Behandlung von Migräne eingesetzt werden können.
Der Gegenstand der vorliegenden Anmeldung unterscheidet sich vom Stand der Technik dadurch, dass ein Verfahren verwendet wird, bei dem die Säureadditionssalze als sphärisch nanostrukturierte Partikel gewonnen werden, ohne dass die Oberfläche der Mikropartikel aus Lezithin besteht.

Das US Patent 6,165,506 offenbart eine feste Darreichungsform von Naproxen in Form von Nanopartikeln, welche verbesserte Auflösungseigenschaften zeigen, wenn ein Oberflächenverbesserer auf der Oberfläche der Partikel adsorbiert wird.

Bekannt in der Literatur sind Inhalationssysteme, in denen der Wirkstoff entweder als mikronisierte Suspension in einem passenden Lösungsmittelsystem als Träger vorliegt oder in Form eines trockenen Pulvers.
Üblicherweise werden Pulverinhalativa z.B. in Form von Kapseln zur Inhalation auf Basis der allgemeinen Lehre, wie sie in DE-A-179 22 07 beschrieben ist, unter Verwendung der chemisch stabilsten Form des Wirkstoffs hergestellt. Dabei werden pharmazeutische Präparate, hergestellt durch Mischen eines fein zerteilten Medikaments mit einem gröberen Trägermedium, in einem Luftstrom durch ein sogenanntes "Staubfließverfahren" unter Ausnutzung der Saugfunktion des Inhalators als Hauptenergiequelle dispergiert.

Ein kritischer Faktor bei solchen Mehrstoffsystemen ist eine gleichmäßige Verteilung des Arzneimittels in der Pulvermischung. Weiterhin ist eine zusätzliche Belastung der Lunge durch den Trägerstoff gegeben sowie das Auftreten von unerwünschten Wechselwirkungen, was zu Kompatibilitätsprobleme führen kann.

Ein bedeutender Aspekt bei der inhalativen Applikation des Wirkstoffes ist, dass nur Teilchen einer bestimmten aerodynamischen Größe in das Zielorgan Lunge gelangen. Die Teilchengröße dieser lungengängigen Partikel (inhalierbarer Anteil) liegt im Submikronbereich. Solche Partikel werden üblicherweise durch Mikronisierung (Luftstrahlmahlung) erzeugt. Daraus ergibt sich oftmals, dass solche Partikel durch diesen mechanischen Schritt hinsichtlich ihrer Kristalleigenschaften komplex zusammengesetzt sein können. Ebenso bedingt die geometrische Form der Partikel des Ausgangsmaterials die morphologischen Eigenschaften des Mikronisats.

Neben dem Strahlmahlverfahren, wobei dem Luftstrahlmahlverfahren besondere Bedeutung zukommt, ist es auch möglich, ein geeignetes Mikronisat über Alternativverfahren herzustellen. Geeignete Mikronisierverfahren zur Herstellung von Mikropartikeln im Submikronbereich sind z.B. das Fällungsverfahren einschließlich der Verfahren, bei denen der Wirkstoff durch Einengen des Lösungsmittels über die maximale Löslichkeit hinaus als nicht-kristalliner Feststoff (amorph) abgeschieden werden kann, die Fällung mittels superkritischer Gase, wie z.B. das RESS- oder das PGSS-Verfahren (J. Jung, M. Perrut: *Particle Design Using Supercritical Fluids,* J. Supercrit. Fluids 20 (2001), 179-219), das GASR-Verfahren (M.P. Gallager et al.: *Gas Antisolvent Recrystallization,* Am. Chem. Soc. (1989)), das PCA-Verfahren (D.J. Dixon, K.P. Johnston: *Polymeric Materials Formed by Precipitation with compressed Fluid Antisolvent,* AIChE Journal (1993, Vol. 39(1), 127), die Gefriertrocknung, die Sprühtrocknung oder eine Kombination aus mehreren der vorstehend genannten Verfahren.

Literaturbekannt ist, dass mittels Sprühtrocknung lungengängige Partikel mit einer Größe zwischen 0.5 µm und 10 µm, bevorzugt zwischen 0.5 µm und 6 µm, hergestellt werden können. Üblicherweise werden aus solchen Sprühtrocknungspartikeln in Anlehnung an oben zitiertes Verfahren (DE-A-179 22 07) technisch handhabbare Formulierungen hergestellt, die eine ausreichende Dispergierbarkeit bei der medizinischen Anwendung (Inhalation) aufweisen [Y.-F. Maa, P.-A. Ngyuyen, J.D. Andya, N. Dasovich, T.D. Sweeny, S.J. Shire, C.C. Hsu, Pharmaceutical Research, 15, No. 5 (1998), 768-775; M.T. Vidgrén, P.A. Vidgrén, T.P. Paronen, Int. J. Pharmaceutics, 35 (1987), 139-144; R.W. Niven, F.D. Lott, A.Y. Ip, J.M. Cribbs, Pharmaceutical Research, 11, No. 8 (1994), 1101-1109].

Neben diesen Beispielen gibt es weitere, vor allem von pharmazeutischen Unternehmen vorgestellte Herstelltechniken auf Basis von Sprühtrocknungsverfahren, die spezielle Formulierungen für Pulverinhalativa beschreiben.

Neben den vorstehend angegebenen Erfordernissen ist generell zu berücksichtigen, dass jede Änderung des Feststoffzustandes eines Arzneimittels, welche dessen physikalische und chemische Stabilität sowie technischen Eigenschaften verbessern kann, gegenüber weniger stabilen Formen desselben Arzneimittels einen erheblichen Vorteil ergibt.

### Problemstellung

Die komplexe Aufgabe der vorliegenden Erfindung bestand primär in der Bereitstellung einer bioverfügbaren Formulierung für die Salze des hochwirksamen CGRP-Antagonisten BIBN4096. Die erfindungsgemäße Formulierung sollte zur Behandlung der akuten, bei Migräne sehr plötzlich eintretenden Schmerzzustände einen schnellen Wirkungseintritt zeigen. Das bedeutet, dass eine rasche Aufnahme des Wirkstoffs und ein schneller Anstieg des Plasmaspiegels gewährleistet sein müssen.

Daher bestand die Aufgabe weiterhin in der Bereitstellung neuartiger, ausreichend stabiler Salze von BIBN4096, die in besonderer Art und Weise zur Herstellung von Pulverinhalativa geeignet sind, welche ohne Zusätze von Trägermaterialien -zur Vermeidung der daraus resultierenden Probleme bei der Verabreichung als Mehrstoffgemisch- direkt einsetzbar sind. Die einzelnen sphärisch nanostrukturierten Mirkopartikel des Pulvers sollten daher sowohl von ihrer Partikelgröße (Aerodynamik) als auch bezüglich ihrer Dispergierbarkeit geeignet sein, inhalativ dargereicht zu werden. Ferner sollte die Formulierung in möglichst wenigen Arbeitsschritten zugänglich und das Pulver technisch bezüglich seiner kohäsiven Eigenschaften ausreichend gut verarbeitbar sein.

### Beschreibung der Erfindung

Ein schneller Wirkungseintritt zur Behandlung von akuten Schmerzzuständen sowie ein hoher Plasmaspiegel der Salze des Wirkstoffs BIBN4096 innerhalb kürzester Zeit lässt sich neben der intravenösen Gabe am besten über die Lunge als Aufnahmeorgan realisieren.

Im Rahmen der vorliegenden Erfindung wurde nun überraschend gefunden, dass die Salze von BIBN4096 durch eine inhalative Darreichung in ausreichendem Maße bioverfügbar gemacht werden können. Es hat sich herausgestellt, dass bei inhalativer Gabe des Wirkstoffs in Form sphärisch nanostrukturierter Mikropartikel eine Bioverfügbarkeit von ca. 60% bezogen auf den Feinanteil der Formulierung (entspricht FPD bestimmt nach USP 24 Suppl. 2000) erreicht werden kann.

Die vorliegende Erfindung bezieht sich auf Säureadditionssalze der Wirkstoffbase 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin [BIBN4096] der Formel (I), bevorzugt sind diejenigen Säureadditionssalze, die ausgewählt sind aus der Gruppe bestehend aus 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin-Hydrochlorid, -Sulfat, -Phosphat, -Hydrobromid, -Carbonat, -Methansulfonat, -*p*-Toluolsulfonat, -Nitrat, -Citrat, -Malat, -Tatrat, -Lactat, -Succinat, -Gluconat, -Acetat, -Formiat, -Propionat, -Capronat, -Oxalat, -Maleat, -Fumarat, -Mandelat und -Hydroxysuccinat, wobei das 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin-Hydrochlorid, das -Sulfat und das -Hydrobromid besonders bevorzugt und das 1-[N²-[3,5-Dibrom-N-[[4-(3,4-dihydro-2(1H)-oxochinazolin-3-yl)-1-piperidinyl]-carbonyl]-D-tyrosyl]-L-lysyl]-4-(4-pyridinyl)-piperazin-Hydrochlorid ganz besonders bevorzugt sind.

Die vorstehend genannten Salze liegen unter Normalbedingungen (T < 50°C, relative Feuchte < 75%) in einem stabilen, amorphen Zustand vor.
Sie können analog literaturbekannten Verfahren hergestellt werden. Beispielsweise lassen sich die Salze durch Lösen der Wirkstoffbase in einem Lösungsmittel wie Wasser oder einem wässrigen Puffersystem mit einem pH-Wert zwischen 4 und 8, bevorzugt zwischen 6 und 8, Zugabe der entsprechenden Säure im Überschuss, bevorzugt durch Zugabe von 0.9 bis 1.1 Äquivalenten der entsprechenden Säure, und anschließendem Entfernen des Lösungsmittels z.B. unter vermindertem Druck mittels eines Rotationsverdampfers, durch Gefriertrocknung, durch Sprühtrocknung oder vergleichbaren Verfahren, bei denen der Lösung das Lösungsmittel entzogen wird, darstellen. Weitere Verfahren sind diejenigen, bei denen die Substanz über eine Anreicherung im Lösungsmittel, wobei eine Übersättigung erreicht wird, als Feststoff gewonnen werden kann.

Ein erster Gegenstand der Erfindung ist somit ein Verfahren zur Herstellung der vorstehend erwähnten Säureadditionssalze, dadurch gekennzeichnet, dass
(a) die Wirkstoffbase in einem Lösungsmittel, ausgewählt aus der Gruppe bestehend aus Wasser und einem wässrigen Puffersystem mit einem pH-Wert zwischen 4 und 8, bevorzugt zwischen 6 und 8, gelöst wird,
(b) die so hergestellte Lösung mit einem Überschuss der Säure, bevorzugt mit 0.9 bis 1.1 Äquivalenten der Säure, versetzt wird und
(c) das Lösungsmittel entfernt wird.

Bei dem vorstehend genannten Verfahren wird das Lösungsmittel bevorzugt mittels Sprühtrocknung entfernt.

Weiterhin wurde gefunden, dass sich die Salze des Wirkstoffs BIBN4096 durch ein ausgezeichnetes Lösungsverhalten von >> 1 mg/ml in Wasser bei einem pH-Wert von 7 auszeichnen. Sie zeichnen sich ebenfalls durch spezielle physikalische und physikochemische Eigenschaften aus, die zu einer verbesserten pharmakologischen/pharmakokinetischen Wirkung bei inhalativer Anwendung der Substanz führen.
Dabei liegen die Vorteile in besonderen pharmakologischen/pharmakokinetischen Eigenschaften bestimmter Salzformen der Wirksubstanz begründet, was dazu führt, dass einhergehend mit der sehr guten Löslichkeit eine umfangreiche und/oder sehr schnelle Verfügbarkeit gegeben ist. Die Verfügbarkeit der Substanz -sowohl quantitativ, bezogen auf die verabreichte Wirkstoffmenge, als auch bezogen auf einen möglichst schnell zu erreichenden hohen Plasmaspiegel- wird neben den biochemischen Eigenschaften der Substanz auch durch physikochemische Eigenschaften bedingt. Sofern ein Feststoff -wie im Falle eines Pulverinhalativumsverabreicht wird, sind im speziellen hierbei die Parameter absolute Löslichkeit im Umgebungsmedium als auch Lösungsgeschwindigkeit im Umgebungsmedium als Funktion der lokalen Konzentration des Wirkstoffes und der Zeit zu berücksichtigen. Während hingegen z.B. für orale Darreichungen, bei denen sich der Wirkstoff bedingt durch die Formulierung möglichst schnell im Umgebungsmedium verteilt, die oben genannten Parameter aufgrund der geringen lokalen Konzentration des Wirkstoffes gegebenenfalls eine untergeordnete Rolle spielen können und somit diese Parameter nicht bzw. nur bedingt durch die Salzform gesteuert werden, sondern üblicherweise mittels spezieller Formulierungen beeinflusst werden, sind diese physikalisch-chemischen Eigenschaften für Pulverinhalativa von großer Bedeutung.

Durch weitere technische Bearbeitung können aus den vorstehend genannten Salzen neuartige, stabile Formulierungen für Pulverinhalativa unter Verzicht eines Trägermaterials hergestellt werden. Prinzipiell können diese Salze durch geeignete Mikronisierverfahren in eine Partikelgröße < 10 µm überführt werden, die dann beispielsweise durch Zumischung von Hilfsstoffen in die geeignete Form (Pulvermischung) gebracht werden, so dass dadurch die technischen Eigenschaften wie Verarbeitbarkeit und Stabilität des Produktes gewährleistet wird. Diese Pulvermischungen können prinzipiell in vordosierten Systemen (z.B. Inhaletten) oder auch in geeigneten Mehrdosissystemen ihre Anwendung finden.

Ein zweites Gegenstand der vorliegenden Erfindung ist die Verwendung eines der vorstehend genannten Säureadditionssalze zur Herstellung des erfindungsgemäßen Pulverinhalativums zur Behandlung von Migräne.

Erfindungsgemäß wurde gefunden, dass die Salze von BIBN4096 durch ein Sprühtrocknungsverfahren überraschenderweise morphologisch so verändert werden können, dass ein derart hergestelltes Pulver ohne weitere Schritte, vor allem ohne die Notwendigkeit des Mischens mit einem gröberen Trägermaterial, direkt in ein Primärpackmittel abgefüllt werden kann und aus diesem heraus mittels eines Pulverinhalationsdevices zur Inhalation ausgebracht werden kann.
Dabei kann das Herstellverfahren so gesteuert werden, dass die Partikel in geeigneter Korngröße vorliegen, üblicherweise zwischen 0.1 und 10 µm und diese Partikel solche Oberflächeneigenschaften besitzen, dass diese leicht verwirbelbar/dispergierbar sind.

Es wurde ferner gefunden, dass die Partikelmorphologie einschließlich der Partikelgröße maßgeblich durch die Wahl der Prozessparameter und Herstellparameter gezielt gesteuert werden kann. Dabei ist es überraschend, dass Pulver dieser Substanz, die mittels "klassischem" Strahlmahlverfahren mikronisiert wurden und in einem vergleichbaren Korngrößenspektrum vorliegen, sich jedoch morphologisch von Partikeln, die gemäß dieser Erfindung hergestellt wurden, bezüglich ihrer Oberflächeneigenschaften/Partikel-Partikel-Wechselwirkungen grundsätzlich unterscheiden. Dies zeigt sich daran, dass sich der Qualitätsparameter "Fine Particle Fraction of Delivered Dose" (z.B. nach Methode zur Bestimmung der "Aerodynamic Particle Size Distribution" - USP 24 oder Pharm. Eur. Suppl. 2000) um den Faktor 10 und mehr verbessert. Da gleichzeitig auf ein Trägermaterial in der Formulierung verzichtet werden kann, verbessert sich bei vorgegebener applizierbarer Pulvergesamtmenge die tatsächlich dem Patienten verfügbare absolute Wirkstoffdosis um einen noch bedeutend höheren Faktor.

Das erfindungsgemäße Herstellverfahren ist dadurch gekennzeichnet, dass der Wirkstoff in geeigneter Weise gelöst, versprüht und in einem Sprühturm getrocknet wird. Das Prinzip der Sprühtrocknung besteht darin, eine Lösung oder Suspension des zu trocknenden Produkts in feine Tröpfchen zu zerteilen und mit einem heißen Gasstrom zu trocknen. Der nach Verdampfen des Lösungsmittels zurückbleibende Feststoffanteil wird aus dem Gasstrom mittels eines Massenkraftabscheiders (z.B.
Zyklon) und/oder durch eine Filtereinheit abgetrennt und gesammelt. Die so hergestellten Mikropartikel zeichnen sich dabei durch spezielle Werte hinsichtlich Partikelgröße, spezifischer Oberfläche und Morphologie aus.

Als geeignete Lösungsmittel haben sich Wasser oder ein wässriges Puffersystem vom pH-Wert zwischen 6 und 8 herausgestellt. Dabei wird der als freie Base vorliegende Wirkstoff erfindungsgemäß in einer wäßrigen Lösung, die entsprechend der zu lösenden Wirkstoffmenge mit 0.9 bis 1.1 Äquivalenten einer Säure versetzt wird, in Form des entsprechenden Salzes in Lösung gebracht. Als Säuren werden dabei erfindungsgemäß bevorzugt anorganische Säuren, wie beispielsweise die Salzsäure, Bromwasserstoffsäure, Phosphorsäure, Salpetersäure, Schwefelsäure, Kohlensäure Methansulfonsäure oder *p*-Toluolsulfonsäure, Fruchtsäuren, wie beispielsweise die Zitronensäure, Äpfelsäure, Weinsäure, Milchsäure, Bernsteinsäure oder Gluconsäure, Carbonsäuren, wie beispielsweise die Ameisensäure, Essigsäure, Propionsäure oder Hexansäure, sowie weitere organische Säuren, wie beispielsweise die Oxalsäure, Fumarsäure, Mandelsäure oder Maleinsäure verwendet, wobei die Verwendung von Salzsäure, Bromwasserstoffsäure oder Schwefelsäure und insbesondere die Verwendung von Salzsäure von besonderer Bedeutung ist.

Die Einstellung der Wirkstoffkonzentration dient primär dazu, den Prozess wirtschaftlich zu gestalten. Dabei sind jedoch der einzustellenden Wirkstoffkonzentration Grenzen gesetzt, die dadurch vorgegeben werden, dass die Oberflächeneigenschaften und die Morphologie der Partikel durch ein bestimmtes Verhältnis zwischen Tropfengröße und Feststoffkonzentration optimiert werden können. Üblicherweise ist eine Konzentration zwischen 0.5 und 25% Gewichtsprozent, in bevorzugter Art und Weise zwischen 1 und 20 Gewichtsprozent, in sehr bevorzugter Art und Weise zwischen 2 und 10 Gewichtsprozent zu wählen. Die Tropfengröße ist ein entscheidender Parameter zur Erzeugung inhalierbarer Partikel. In Abhängigkeit der verwendeten Düse ist der Sprühgasdurchsatz in Kombination mit dem Lösungsdurchsatz so zu wählen, dass die gewünschte Tropfengröße erzielt wird. Da es eine Vielzahl von Parameter-Kombinationen Düse-Sprühgasdurchsatz-Lösungsdurchsatz gibt, die zu einer geeigneten Tropfengröße führen, wird eine sinnvolle Spezifizierung des Verfahrens über die Tropfengröße getroffen, welche beim Prozess gewählt werden soll. Diese kann durch den Kennwert X₅₀ (Medianwert = Teilchengröße/Tropfengröße, unterhalb derer 50% der Teilchenmenge liegt bezüglich der Volumenverteilung der einzelnen Teilchen/Tropfen), der im Bereich zwischen 1.5 µm und 30 µm, bevorzugt zwischen 1.5 µm und 20 µm, besonders bevorzugt zwischen 1.5 µm und 8 µm, liegen sollte, sowie den Kennwert Q_{(5.8)} (entspricht der Teilchenmenge, die bezogen auf die Volumenverteilung der Tröpfchen unterhalb von 5.8 µm liegt), der zwischen 10% und 100%, bevorzugt zwischen 20% und 100% und besonders bevorzugt zwischen 50% und 100% liegen sollte, charakterisiert werden.

Technisch umgesetzt wird dies, in dem eine entsprechende kommerzielle Düse, z. B. Ein- oder Mehrstoffdüsen, die in Abhängigkeit der Düsenparameter (z. B. Rotationsgeschwindigkeit bei Rotationszerstäubern oder angesetztem Zerstäubungsdruck und des daraus resultierenden Massestroms des Zerstäubungsgases bei Zweistoffdüsen) sowie der Spray-Rate (Volumenstrom "Sprühlösung") diese Charakteristika aufweist, eingesetzt wird. Neben den besonderen Bedingungen, die im eigentlichen Sprühprozess eingehalten werden müssen, um geeignete Tröpfchen für den Trocknungsprozess zu generieren, zeigt sich, dass die Oberflächeneigenschaften der Partikel auch durch die Wahl der Trocknungsparameter positiv/gezielt beeinflusst werden können. Die entscheidenden Kenngrößen, die in den Trocknungsschritt einfließen, sind Eingangs- und Ausgangstemperatur des Trocknungsgases, sowie der Volumenstrom des durchgesetzten, Trockengases.
Es ist zu beachten, dass die Tropfen mit geeigneter Tropfengröße so durch die Trocknungskammer geführt werden, dass die Tröpfchen und die getrockneten Partikel nicht oder nur geringfügig in Berührung mit der Wand des Sprühturms kommen. Dies wird durch Düsen mit entsprechendem Sprühkegel, durch einen Sprühturm mit geeignetem Durchmesser und durch die Strömungsbedingungen in der Apparatur erzielt. Die Ausgangstemperatur muss für den Prozess so angepasst werden, dass das Pulver einen ausreichend geringen Restlösemittelgehalt aufweist und somit eine ausreichende chemische und physikalische Stabilität erreicht wird. Diese ist idealerweise gegeben, wenn die Ausgangs-Temperatur im Bereich der Siedetemperatur des Lösungsmittels bzw. gering darüber gehalten wird. Dagegen ist die Einlasstemperatur des Trocknungsgases so zu wählen, dass in Kombination mit dem Parameter Volumenstrom "Trocknungsgas" sowie Spray-Rate das Trocknen so schonend abläuft, dass Partikel mit geeigneten Oberflächeneigenschaften entstehen.

Ein drittes Gegenstand der Erfindung ist somit ein Verfahren zur Herstellung eines Pulverinhalativums, umfassend die Schritte
(a) Lösen des Wirkstoffs BIBN4096 in Wasser oder einem wäßrigen Puffersystem mit einem pH-Wert zwischen 4 und 8, bevorzugt zwischen 6 und 8, enthaltend entsprechend der zu lösenden Wirkstoffmenge einen Überschuss, bevorzugt 0.9 bis 1.1 Äquivalente, einer Mineralsäure oder einer organischen Säure, wobei der Wirkstoff in das entsprechende Salz übergeführt wird,
(b) Versprühen der so gewonnenen Wirkstofflösung auf übliche Weise, so dass ein Sprühnebel mit einer Tropfengröße mit dem Kennwert X₅₀ im Bereich von 1.5 bis 30 µm, bevorzugt von 1.5 bis 20 µm, besonders bevorzugt von 1.5 bis 8 µm, und Q_{(5.8)} zwischen 10% und 100%, bevorzugt zwischen 20% und 100%, besonders bevorzugt zwischen 50% und 100%, erzielt wird,
(c) Trocknen des so erhaltenen Sprühnebels mit Hilfe eines Trocknungsgases unter Anwendung folgender Parameter:
   - einer Eingangstemperatur des Trocknungsgases von 100 °C bis 350 °C, bevorzugt zwischen 120 °C und 250 °C und besonders bevorzugt zwischen 130 °C und 200 °C,
   - einer Ausgangstemperatur des Trocknungsgases von 40 °C bis 120 °C (vgl. Beispiel),
   - einem Volumenstrom des Sprühgases von 1 Nm³/h bis 15 Nm³/h,
   - einem Volumenstrom des Trocknungsgases von 15 Nm³/h bis 1500 Nm³/h, bevorzugt von 15 Nm³/h bis 150 Nm³/h, und
(d) Abtrennen des getrockneten Feststoffanteils aus dem Trockungsgasstrom auf übliche Weise.

Die erfindungsgemäß dargestellten Partikel weisen eine hohe physikalische Stabilität auf. Insbesondere ermöglichen die Partikeleigenschaften bei der Anwendung als Pulverinhalativum einen hohen Feinanteil bei der Ausbringung, technisch bestimmt z.B. mittels Kaskadenimpaktormessung (Andersenkaskaden-Impaktor, nach USP 24 bzw. Pharm. Eur. Suppl. 2000). Typischerweise ist der Anteil der Partikel nach dieser Methode, die kleiner als 5 µm (aerodynamisch) sind, größer 15%, es werden dabei zum Teil sogar Feinanteile von mehr als 50% erreicht. Neben diesem Key-Parameter für Inhalativa zeichnet sich das Pulver dadurch aus, dass es sich mit gängigen technischen Verfahren weiterverarbeiten lässt. Gekennzeichnet sind so hergestellte Pulver durch die physikochemischen Parameter Partikelgröße, z.B. gemessen mittels Laserbeugung, sowie spezifische Oberfläche, z.B. gemessen mittels Mehrpunkt B.E.T. Messung. Für den Kennwert Q_{(5.8)} liegt die Partikelgröße so hergestellter Pulver typischerweise zwischen 50% und 100% sowie für den Parameter X₅₀ zwischen 1 µm und 6 µm. Partikel, die nach obigen Verfahren hergestellt sind, weisen dabei typischerweise Werte für die Spezifische Oberfläche zwischen 1 m²/g und 20 m²/g, idealerweise zwischen 1 m²/g und 10 m²/g auf. Geometrisch weisen Partikel, die nach obigen Verfahren hergestellt werden, Partikelformen auf, die je nach Versuchsbedingung zwischen den Extrema "Kugelform", "Kugelform mit Hohlraum, evtl. mit Loch", "Kugelform mit nach innen-geformten Wölbungen", sowie "zusammengefallene Hohlkörper" beschrieben werden können. Rasterelektronenmikroskopisch ist die Oberfläche solcher Partikel weitestgehend glatt bzw. an der Oberfläche (sphärisch) nanostrukturiert.

Ein viertes Gegenstand der vorliegenden Erfindung ist somit ein Pulverinhalativum, enthaltend eines der vorstehend erwähnten Säureadditionssalze der Wirkstoffbase 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin [BIBN4096] der Formel (I) in Form von Mikropartikeln, dadurch gekennzeichnet, dass
(a) die Partikel eine spezifische Oberfläche zwischen 1 m²/g und 20 m²/g aufweisen, bevorzugt zwischen 1 m²/g und 10 m²/g,
(b) der Kennwert Q_{(5.8)} zwischen 50% und 100 % und
(c) der Parameter X₅₀ zwischen 1 µm und 6 µm liegt.

Ein funftes Gegenstand der vorliegenden Erfindung ist ein Pulverinhaltivum, erhältlich nach einem voranstehend genannten erfindungsgemäßen Verfahren.

### Experimenteller Teil

### 1) Meßverfahren

### a) Bestimmung der Partikelgröße mittels Laserbeugung (Frauenhoferbeugung):

- Meßmethode:: Zur Bestimmung der Partikelgröße wird das Pulver mittels Dispergiereinheit einem Laserbeugungs-Spektrometer zugeführt. Unter dem Medianwert X₅₀ versteht man die Teilchengröße, unterhalb derer 50% der Teilchenmenge liegt. Der Q_{(5,8)} - Wert beschreibt den prozentualen Anteil der Teilchen, die eine Größe unterhalb von 5.8 µm aufweisen.
- Meßgerät:: Laser-Beugungs-Spektrometer (HELOS),Fa. Sympatec
- Software:: WINDOX 4
- Dispergiereinheit:: RODOS / Dispergierdruck: 3 bar
- Brennweite:: 100 mm [Meßbereich: 0.9.....175µm]
- Auswertemodus:: HRLD (V 4)

### b) Bestimmung der Spezifischen Oberfläche:

- Meßmethode:: Die Bestimmung der spezifischen Oberfläche erfolgt, indem die Pulverprobe einer Stickstoffatmosphäre bei unterschiedlichen Drücken ausgesetzt wird. Durch Abkühlung der Probe erfolgt eine Kondensation der Stickstoffmolekülen auf der Oberfläche der Partikel. Die kondensierte Stickstoffmenge wird über den Druckabfall im System bestimmt und die spezifische Oberfläche der Probe über den Flächenbedarf von Stickstoff und der Probeneinwaage berechnet.
- Meßgerät: Tri Star Multi Point BET, Fa. Micromeritics
- Ausheizstation:: VacPrep 061, Fa. Micromeritics
- Ausheizen:: ca. 12 h / 40 °C

### Analysenparameter

- Probengefäß:: 0.0127 m (½ inch); mit "filler rod"
- Analyseverfahren:: 16 Punkt BET Oberflächenbestimmung 0.05 bis 0.20 p/p0
- absolute Druck-Toleranz:: 666.6 Pa (5.0 mm Hg)
- relative Druck-Toleranz:: 5.0%
- Evakuierungs-Geschwindigkeit:: 6666.1 Pa/Sekunde (50.0 mm Hg/Sekunde)
- Evakuierungsschwellenwert:: 1333.3 Pa (10.0 mm Hg)
- Evakuierungsdauer:: 0.1 h
- Leervolumen:: Dewargefäß-Absenkung, t: 0.5 h
- Haltezeit:: 20 Sekunden
- Minimale Gleichgewichtseinstellungsdauer:: 600 Sekunden
- Adsorbens:: Stickstoff

### c) Bestimmung der Tropfengröße mittels Laserbeugung (nach Mie):

- Meßgerät:: Laser-Beugungs-Spektrometer (HELOS), Fa. Sympatec
- Software:: WINDOX 4
- Brennweite:: 100 mm [Meßbereich: 0,9.....175 µm]
- Meßmethode:: Die Ermittlung der Tropfengröße erfolgt, indem die Düse aus dem Sprühtrockner herausgenommen wird und der Spray im oberen Drittel des Sprühkegels zentrisch in den Laserstrahl gebracht wird. Die Messung erfolgt bei Raumtemperatur mit Wasser als Referenzmedium unter ansonsten gleichen Bedingungen.

### 2) Beispiele für Sprühparameter

### Beispiel 1: Sprühparameter, geeignet für eine wäßrige BIBN4096-Lösung (modifizierter BÜCHI-Sprühturm):

| | | |
|---|---|---|
| Konzentration Lösung | | 10 g BIBN4096 in 100 ml einer 0.115 mol / L HCl |
| Tröpfchengröße | Q_{(5.8)} | 71 % |
| (Referenzlösung: H₂O bei Raumtemperatur) | X₅₀ | 3.9 µm |
| Volumenstrom "Sprührate" | | 9 ml/min |
| Sprühdruck *(Düsentyp)* | | 4.6 bar Überdruck (N₂) |
| | | *(BÜCHI Sprühdüse 0.7 mm, Art.-Nr. 04364)* |
| Volumenstrom "Zerstäubungsdruck" | | 2211 Normliter / h |
| (Düsentyp) | | *(BÜCHI Sprühdüse 0.7 mm, Art.-Nr. 04364)* |
| Eingangstemperatur | | 150 °C |
| Ausgangstemperatur | | 100 °C |
| Volumenstrom "Trocknungsgas" | | 30 Norm m³ / h |
| Querschnitt Trockenturm | | 105 mm |

### Beispiel 2: Sprühparameter, geeignet für eine wäßrige BIBN4096-Lösung (modifizierter BÜCHI-Sprühturm):

| | | |
|---|---|---|
| Konzentration Lösung | | 9.1 g/100g |
| Lösungsmittel: | | 0.115 mol / L HCl |
| Tröpfchengröße | Q_{(5.8)} | < 10% |
| (Referenzlösung: H₂O bei Raumtemperatur) | X₅₀ | 13 µm |
| Volumenstrom "Sprührate" | | 0.54 L/h |
| Sprühdruck (*Düsentyp*) | | 1.0 bar Überdruck (N₂) |
| | | *(BÜCHI Sprühdüse 0.7 mm, Art.-Nr. 04364)* |
| Massenstrom Sprühgas Düsentyp | | 0.72 kg / h |
| | | *(BÜCHI Sprühdüse 0.7 mm, Art.-Nr. 04364)* |
| Eingangstemperatur | | 150 °C |
| Ausgangstemperatur | | 104 °C |
| Volumenstrom ''Trocknungsgas'' | | 35-36 Norm m³ / h |
| Querschnitt Trockenturm | | 105 mm |

### Beispiel 3: Sprühparameter, geeignet für eine wäßrige BIBN4096-Lösung (modifizierter BÜCHI-Sprühturm):

| | | |
|---|---|---|
| Konzentration Lösung | | 4.0 g/100g |
| Lösungsmittel: | | 0.05 mol / L HCl |
| Tröpfchengröße | Q_{(5.8)} | 69% |
| (Referenzlösung: H₂O bei Raumtemperatur) | X₅₀ | 5 µm |
| Volumenstrom "Sprührate" | | 0.63 L/h |
| Sprühdruck | | 6.1 bar Überdruck (N₂) |
| *(Düsentyp)* | | *(BÜCHI Sprühdüse 0.7 mm, Art.-Nr.* 04364) |
| Massenstrom Sprühgas | | 3.4 kg/h |
| (Düsentyp) | | *(BÜCHI Sprühdüse 0.7 mm, Art.-Nr. 04364)* |
| Eingangstemperatur | | 150 °C |
| Ausgangstemperatur | | 98-99 °C |
| Volumenstrom "Trocknungsgas" | | 35 Norm m³/h |
| Querschnitt Trockenturm | | 105 mm |

### Beispiel 4: Sprühparameter, geeignet für eine wäßrige BIBN4096-Lösung (modifizierter BÜCHI-Sprühturm):

| | | |
|---|---|---|
| Konzentration Lösung | | 10.3 g/100g |
| Lösungsmittel: | | 0.13 mol / L HCl |
| Tröpfchengröße | Q_{(5.8)} | 69% |
| (Referenzlösung: H₂O bei Raumtemperatur) | X₅₀ | 5 µm |
| Volumenstrom "Sprührate" | | 0.63 L / h |
| Sprühdruck | | 6.1-6.2 bar Überdruck (N₂) |
| *(Düsentyp)* | | *(BÜCHI Sprühdüse 0.7 mm, Art.-Nr. 04364)* |
| Massenstrom Sprühgas Düsentyp | | 3.4 kg/h |
| | | *(BÜCHI Sprühdüse 0. 7 mm, Art.-Nr. 04364)* |
| Eingangstemperatur | | 150 °C |
| Ausgangstemperatur | | 98-101 °C |
| Volumenstrom "Trocknungsgas" | | 35 Norm m³/h |
| Querschnitt Trockenturm | | 105 mm |

### Beispiel 5: Sprühparameter, geeignet für eine wäßrige BIBN4096-Lösung (modifizierter BÜCHI-Sprühturm):

| | | |
|---|---|---|
| Konzentration Lösung | | 2 g/100g |
| Lösungsmittel: | | 0.012 mol / L Schwefelsäure |
| Tröpfchengröße | Q_{(5.8)} | 39% |
| (Referenzlösung: H₂O bei Raumtemperatur) | X₅₀ | 7.0 µm |
| Volumenstrom "Sprührate" | | 0.54 L / h |
| Sprühdruck | | 3.3-3.5 bar Überdruck (N₂) |
| (*Düsentyp*) | | *(BÜCHI Sprühdüse 0.7 mm, Art.-Nr. 04364)* |
| Massenstrom Sprühgas Düsentyp | | 2.1 kg/h |
| | | *(BÜCHI Sprühdüse 0.7 mm, Art.-Nr.* 04364) |
| Eingangstemperatur | | 150 °C |
| Ausgangstemperatur | | 101 °C |
| Volumenstrom "Trocknungsgas" | | 35 Norm m³ / h |
| Querschnitt Trockenturm | | 105 mm |

### Beispiel 6: Sprühparameter, geeignet für eine wäßrige BIBN4096-Lösung (modifizierter BÜCHI-Sprühturm):

| | | |
|---|---|---|
| Konzentration Lösung | | 5g/100g |
| Lösungsmittel: | | 0,02 mol / L Citronensäure |
| Tröpfchengröße | Q_{(5.8)} | 55% |
| (Referenzlösung: H₂O bei Raumtemperatur) | X₅₀ | 5.5 µm |
| Volumenstrom "Sprührate" | | 0.54 L / h |
| Sprühdruck | | 4.4-4.6 bar Überdruck (N₂) |
| *(Düsentyp)* | | *(BÜCHI Sprühdüse 0.7 mm, Art.-Nr. 04364)* |
| Massenstrom Sprühgas Düsentyp | | 2.8 kg/h |
| | | *(BÜCHI Sprühdüse 0.7 mm, Art.-Nr. 04364)* |
| Eingangstemperatur | | 150 °C |
| Ausgangstemperatur | | 99 °C |
| Volumenstrom "Trocknungsgas" | | 35 Norm m³ / h |
| Querschnitt Trockenturm | | 105 mm |

### Beispiel 7: Sprühparameter, geeignet für eine wäßrige BIBN4096-Lösung (modifizierter BÜCHI-Sprühturm):

| | | |
|---|---|---|
| Konzentration Lösung | | 10 g/100g |
| Lösungsmittel: | | 0.06 mol / L Weinsäure |
| Tröpfchengröße | Q_{(5.8)} | 60% |
| (Referenzlösung: H₂O bei Raumtemperatur) | X₅₀ | 5.3 µm |
| Volumenstrom "Sprührate" | | 0.54 L/h |
| Sprühdruck | | 4.7-4.9 bar Überdruck (N₂) |
| (*Düsentyp*) | | *(BÜCHI Sprühdüse 0. 7 mm, Art.-Nr. 04364)* |
| Massenstrom Sprühgas Düsentyp | | 3 kg/h |
| | | *(BÜCHI Sprühdüse 0.7 mm, Art.-Nr.04364)* |
| Eingangstemperatur | | 150 °C |
| Ausgangstemperatur | | 101 °C |
| Volumenstrom "Trocknungsgas" | | 35 Norm m³ / h |
| Querschnitt Trockenturm | | 105 mm |

### Beispiel 8: Sprühparameter, geeignet für eine wäßrige BIBN4096-Lösung (modifizierter BÜCHI-Sprühturm):

| | | |
|---|---|---|
| Konzentration Lösung | | 20 g/100g |
| Lösungsmittel: | | 0.26 mol / L HBr |
| Tröpfchengröße | Q_{(5.8)} | 69% |
| (Referenzlösung: H₂O bei Raumtemperatur) | X₅₀ | 5.1 µm |
| Volumenstrom "Sprührate" | | 0.54 L / h |
| Sprühdruck | | 6 bar Überdruck (N₂) |
| (*Düsentyp)* | | (*BÜCHI Sprühdüse 0.7 mm, Art.-Nr. 04364)* |
| Massenstrom Sprühgas Düsentyp | | 3.4 kg / h |
| | | (*BÜCHI Sprühdüse 0.7 mm, Art.-Nr. 04364)* |
| Eingangstemperatur | | 150 °C |
| Ausgangstemperatur | | 100 °C |
| Volumenstrom "Trocknungsgas" | | 35 Norm m³/h |
| Querschnitt Trockenturm | | 105 mm |

### Beispiel 9: Sprühparameter, geeignet für eine wäßrige BIBN4096-Lösung (modifizierter BÜCHI-Sprühturm):

| | | |
|---|---|---|
| Konzentration Lösung | | 5g/100g |
| Lösungsmittel: | | 0.06 mol / L Methansulfonsäure |
| Tröpfchengröße | Q_{(5.8)} | 69% |
| (Referenzlösung: H₂O bei Raumtemperatur) | X₅₀ | 5.1 µm |
| Volumenstrom "Sprührate" | | 0.54 L/h |
| Sprühdruck | | 6 bar Überdruck (N₂) |
| *(Düsentyp)* | | (*BÜCHI Sprühdüse 0.7 mm, Art.-Nr. 04364)* |
| Massenstrom Sprühgas Düsentyp | | 3.4 kg/h |
| | | *(BÜCHI Sprühdüse 0.7 mm, Art.-Nr. 04364)* |
| Eingangstemperatur | | 150 °C |
| Ausgangstemperatur | | 100 °C |
| Volumenstrom "Trocknungsgas" | | 35 Norm m³ / h |
| Querschnitt Trockenturm | | 105 mm |

### 3) Charakterisierung der erhaltenen Feststoffpartikel aus einigen der oben genannten Beispiele:

### Beispiel 1:

| | | |
|---|---|---|
| Partikelgröße | X₅₀ | 2.5 µm |
| | Q_{(5.8)} | 97% |

### Beispiel 2:

| | | |
|---|---|---|
| Partikelgröße | X₅₀ | 5.5 µm |
| | Q_{(5.8)} | 53.3% |
| Spezifische Oberfläche | Sₘ | 1.9 m²/g |

### Beispiel 3:

| | | |
|---|---|---|
| Partikelgröße | X₅₀ | 2.4 µm |
| | Q_{(5.8)} | 96.7% |
| Spezifische Oberfläche | Sₘ | 5.0 m²/g |

### Beispiel 4:

| | | |
|---|---|---|
| Partikelgröße | X₅₀ | 3.0 µm |
| | Q_{(5.8)} | 90.0% |
| Spezifische Oberfläche | Sₘ | 3.4 m²/g |

### Kurzbeschreibung der Abbildungen

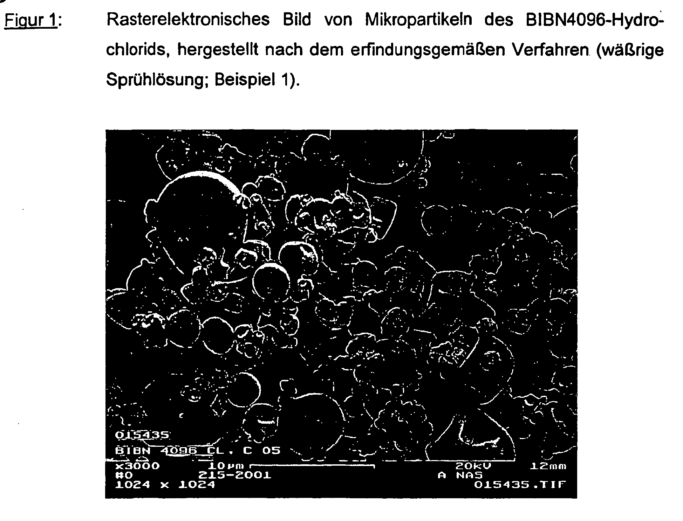

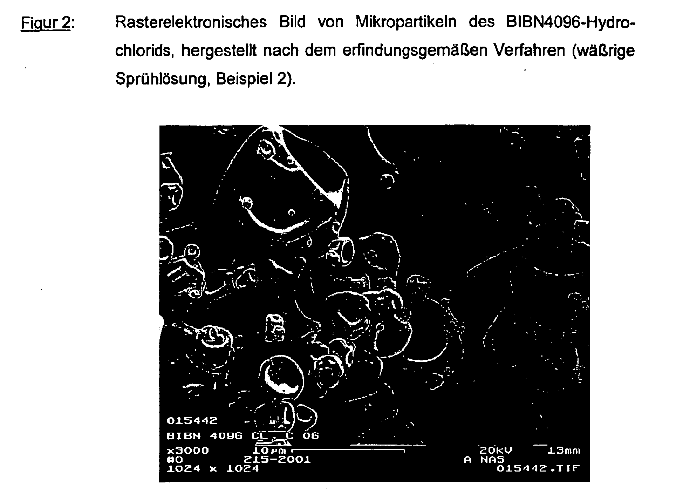

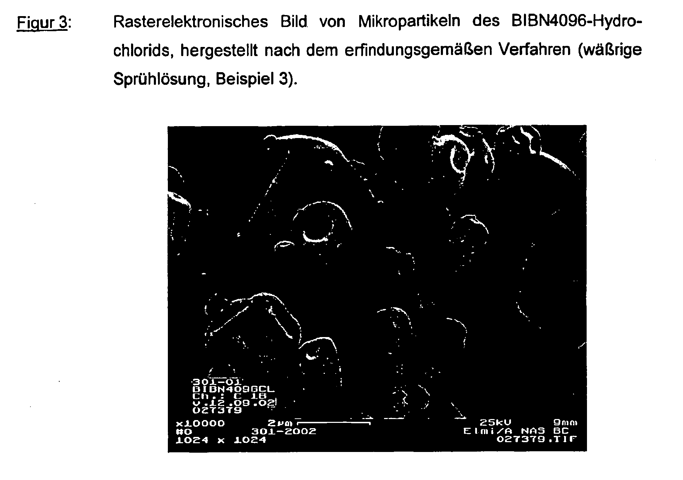

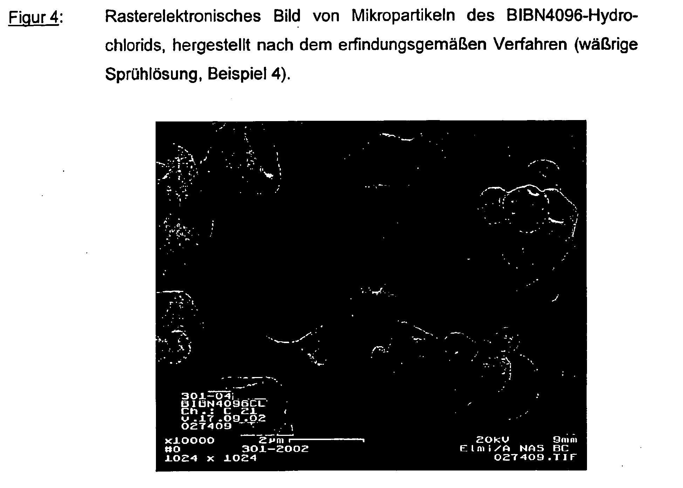

Figuren 1 bis 4 zeigen rasterelektronische Aufnahmen von Mikropartikeln, welche aus einigen der oben genannten Beispiele nach dem erfindungsgemäßen Verfahren aus einer wässrigen Sprühlösung hergestellt wurden.

## Patentansprüche

1. Verfahren zur Herstellung eines Pulverinhalativums, umfassend die Schritte
(a) Lösen eines Säureadditionssalzes der Wirkstoffbase 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin [BIBN4096] der Formel 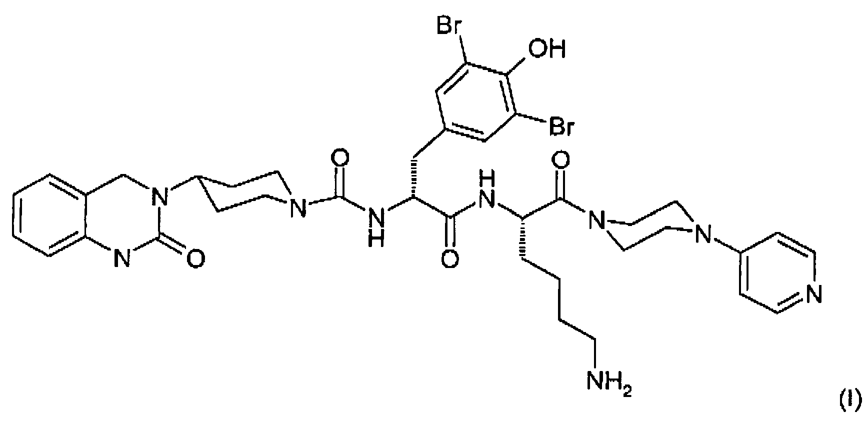 oder Lösen des Wirkstoffs BIBN4096 in einem wäßrigen System, enthaltend entsprechend der zu lösenden Wirkstoffmenge einen Überschuss, bevorzugt 0.9 bis 1.1 Äquivalente, einer Mineralsäure oder einer organischen Säure, wobei der Wirkstoff in das entsprechende Salz übergeführt wird,
(b) Versprühen der so gewonnenen Wirkstofflösung auf übliche Weise, so dass ein Sprühnebel entsteht, bei dem 50% der erhaltenen Teilchenmenge bezüglich der Volumenverteilung der Tropfen (dem Kennwert X₅₀) bei einer Tropfengröße von 1.5 bis 30 µm, bevorzugt von 1.5 bis 20 µm, besonders bevorzugt von 1.5 bis 8 µm liegt, und die Teilchenmenge, die bezogen auf die Volumenverteilung der Tröpfchen unterhalb von 5.8 µm liegt (Kennwert Q_{(5.8)}), zwischen 10% und 100%, bevorzugt zwischen 20% und 100%, besonders bevorzugt zwischen 50% und 100% liegt,
(c) Trocknen des so erhaltenen Sprühnebels mit Hilfe eines Trocknungsgases unter Anwendung folgender Parameter:
• einer Eingangstemperatur des Trocknungsgases von 100 °C bis 350 °C, bevorzugt zwischen 120 °C und 250 °C und besonders bevorzugt zwischen 130 °C und 200 °C,
• einer Ausgangstemperatur des Trocknungsgases von 40 °C bis 120 °C,
• einem Volumenstrom des Sprühgases von 1 Nm³/h bis 15 Nm³/h und
• einem Volumenstrom des Trocknungsgases von 15 Nm³/h bis 1500 Nm³/h, bevorzugt von 15 Nm³/h bis 150 Nm³/h, und
(d) Abtrennen des getrockneten Feststoffanteils aus dem Trockungsgasstrom auf übliche Weise.

2. Verfahren nach Anspruch 1, **dadurch gekennzeichnet, dass** die zur Sprühtrocknung verwendete Wirkstofflösung eine Konzentration von 0.5 bis 25 Gew.-% aufweist.

3. Verfahren nach Anspruch 1, **dadurch gekennzeichnet, dass** die zur Sprühtrocknung verwendete Wirkstofflösung eine Konzentration von 1 bis 20 Gew.-% aufweist.

4. Verfahren nach Anspruch 1, **dadurch gekennzeichnet, dass** die zur Sprühtrocknung verwendete Wirkstofflösung eine Konzentration von 2 bis 10 Gew.-% aufweist.

5. Verfahren nach einem der Ansprüche 1 bis 4, **dadurch gekennzeichnet, dass** das Säureadditionssalz ausgewählt ist aus der Gruppe bestehend aus 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin-Hydrochlorid, -Sulfat, -Phosphat, -Hydrobromid, -Carbonat, -Methansulfonat, -Nitrat, -Citrat, -Malat, -Tatrat, -Lactat, -Succinat, -Gluconat, -Acetat, -Formiat, -Propionat, -Capronat, -Oxalat, -Maleat, -Fumarat, -Mandelat, -Hydroxysuccinat und -*p*-Toluolsulfonat.

6. Verfahren nach einem der Ansprüche 1 bis 4, **dadurch gekennzeichnet, dass** das Säureadditionssalz ausgewählt ist aus der Gruppe bestehend aus 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin-Hydrochlorid, -Sulfat und Hydrobromid.

7. Verfahren nach einem der Ansprüche 1 bis 4, **dadurch gekennzeichnet, dass** das Säureadditionssalz ausgewählt ist aus der Gruppe bestehend aus 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin-Hydrochlorid.

8. Pulverinhalativum, bestehend aus einem Säureadditionssalz der Wirkstoffbase 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin [BIBN4096] der Formel 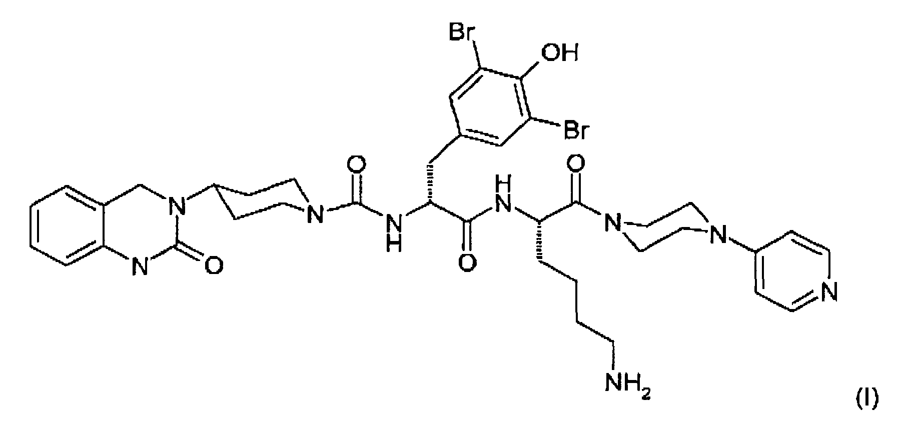 in Form von sphärisch nanostrukturierten Partikeln, **dadurch gekennzeichnet, dass**
(a) die Partikel eine spezifische Oberfläche zwischen 1 m²/g und 20 m²/g aufweisen,
(b) die Teilchenmenge, die bezogen auf die Volumenverteilung der Tröpfchen unterhalb von 5.8 µm (der Kennwert Q_{(5.8)}) liegt, zwischen 50% und 100% und
(c) die Teilchengröße, unterhalb derer 50% der Teilchenmenge bezüglich der Volumenverteilung der einzelnen Teilchen liegt (der Parameter X₅₀), zwischen 1 µm und 6 µm liegt.

9. Pulverinhalativum gemäß Anspruch 8, **dadurch gekennzeichnet, dass** die Partikel eine spezifische Oberfläche zwischen 1 m²/g und 10 m²/g aufweisen.

10. Pulverinhalativum gemäß einem der Ansprüche 8 oder 9, **dadurch gekennzeichnet, dass** das Säureadditionssalz ausgewählt ist aus der Gruppe bestehend aus 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin-Hydrochlorid, -Sulfat, -Phosphat, -Hydrobromid, -Carbonat, -Methansulfonat, -Nitrat, -Citrat, -Malat, -Tatrat, -Lactat, -Succinat, -Gluconat, -Acetat, -Formiat, -Propionat, -Capronat, -Oxalat, -Maleat, -Fumarat, -Mandelat, -Hydroxysuccinat und -*p*-Toluolsuffonat.

11. Pulverinhalativum gemäß einem der Ansprüche 8 oder 9, **dadurch gekennzeichnet, dass** das Säureadditionssalz ausgewählt ist aus der Gruppe bestehend aus 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin-Hydrochlorid, -Sulfat und Hydrobromid.

12. Pulverinhalativum gemäß einem der Ansprüche 8 oder 9, **dadurch gekennzeichnet, dass** das Säureadditionssalz 1-[N²-[3,5-Dibrom-N-[[4-(3,4-dihydro-2(1H)-oxo-chinazolin-3-yl)-1-piperidinyl]carbonyl]-D-tyrosyl]-L-lysyl]-4-(4-pyridinyl)-piperazin-Hydrochlorid ist.

13. Pulverinhalativum gemäß einem der Ansprüche 8 bis 12, erhältlich nach einem Verfahren gemäß einem der Ansprüche 1 bis 7.

## Claims

1. Process for preparing an inhalable powder, comprising the steps of
(a) dissolving an acid addition salt of the active substance base 1-[*N*²-[3,5-dibromo-*N*-[[4-(3,4-dihydro-2(1*H*)-oxoquinazolin-3-yl)-1-piperidinyl] carbonyl]-D-tyrosyl]-L-lysyl]-4-(4-pyridinyl)-piperazine [BIBN4096] of formula 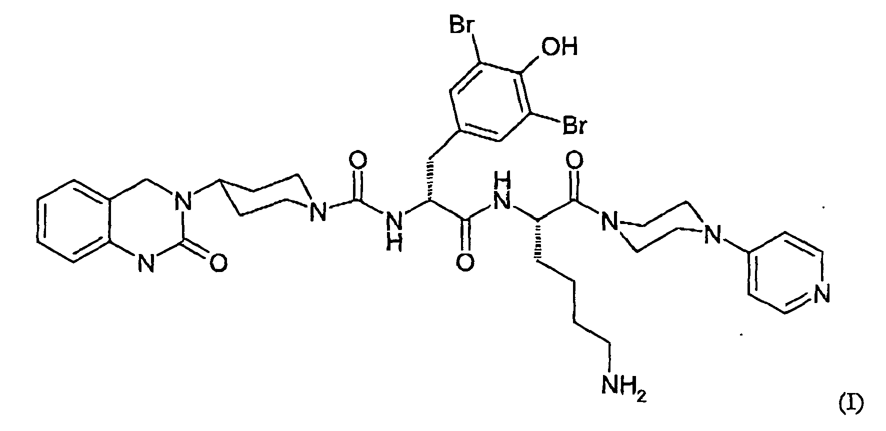 or
dissolving the active substance BIBN4096 in an aqueous system containing an excess, preferably 0.9 to 1.1 equivalents, of a mineral acid or an organic acid, depending on the quantity of active substance which is to be dissolved, whereby the active substance is converted into the corresponding salt,
(b) spraying the resulting solution of active substance in conventional manner so as to obtain a spray mist in which 50% of the quantity of particles contained therein relative to the volume distribution of the droplets (the characteristic value X₅₀) has a droplet size of from 1.5 to 30 µm, preferably from 1.5 to 20 µm, particularly preferably from 1.5 to 8 µm, and the quantity of particles which is below 5.8 µm (characteristic value Q_{(5.8)}) relative to the volume distribution of the droplets is between 10% and 100%, preferably between 20% and 100%, particularly preferably between 50% and 100%,
(c) drying the spray mist thus obtained by means of a drying gas, while applying the following parameters:
• an inlet temperature of the drying gas of 100°C to 350°C, preferably between 120°C and 250°C and particularly preferably between 130°C and 200°C,
• an outlet temperature of the drying gas of 40°C to 120°C,
• a volumetric flow of the spray gas of 1 Nm³/h to 15 Nm³/h, and
• a volumetric flow of the drying gas of 15 Nm³/h to 1500 Nm³/h, preferably of 15 Nm³/h to 150 Nm³/h, and
(d) separating the dried solid fraction from the drying gas current in the conventional manner.

2. Process according to claim 1, **characterised in that** the active substance solution used for the spray-drying has a concentration of 0.5 to 25% by weight.

3. Process according to claim 1, **characterised in that** the active substance solution used for the spray-drying has a concentration of 1 to 20% by weight.

4. Process according to claim 1, **characterised in that** the active substance solution used for the spray-drying has a concentration of 2 to 10% by weight.

5. Process according to one of claims 1 to 4, **characterised in that** the acid addition salt is selected from the group consisting of 1-[*N*²-[3,5-dibromo-*N-*[[4-(3,4-dihydro-2(1*H*)-oxoquinazolin-3-yl)-1-piperidinyl] carbonyl]-D-tyrosyl]-L-lysyl]-4-(4-pyridinyl)-piperazine hydrochloride, sulphate, phosphate, hydrobromide, carbonate, methanesulphonate, nitrate, citrate, malate, tartrate, lactate, succinate, gluconate, acetate, formate, propionate, capronate, oxalate, maleate, fumarate, mandelate, hydroxysuccinate and *p*-toluenesulphonate.

6. Process according to one of claims 1 to 4, **characterised in that** the acid addition salt is selected from the group consisting of 1-[*N*²-[3,5-dibromo-*N-*[[4-(3,4-dihydro-2(1*H*)-oxoquinazolin-3-yl)-1-piperidinyl]carbonyl]-D-tyrosyl]-L-lysyl]-4-(4-pyridinyl)-piperazine-hydrochloride, sulphate and hydrobromide.

7. Process according to one of claims 1 to 4, **characterised in that** the acid addition salt is selected from the group consisting of 1-[*N*²-[3,5-dibromo-*N-*[[4-(3,4-dihydro-2(1*H*)-oxoquinazolin-3-yl)-1-piperidinyl]carbonyl]-D-tyrosyl]-L-lysyl]-4-(4-pyridinyl)-piperazine hydrochloride.

8. Inhalable powder consisting of an acid addition salt of the active substance base 1-[*N*²-[3,5-dibromo-*N*-[[4-(3,4-dihydro-2(1*H*)-oxoquinazolin-3-yl)-1-piperidinyl]carbonyl]-D-tyrosyl]-L-lysyl]-4-(4-pyridinyl)-piperazine [BIBN4096] of formula 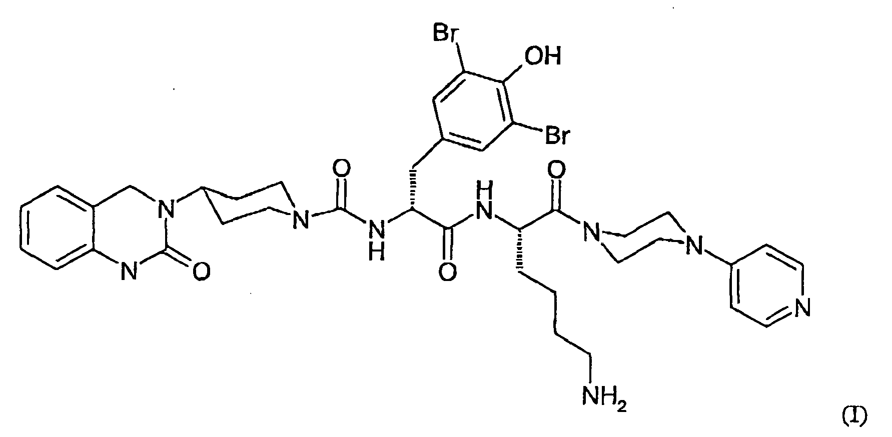 in the form of spherically nanostructured particles, **characterised in that**
(a) the particles have a specific surface of between 1 m²/g and 20 m²/g,
(b) the quantity of particles which is below 5.8 µm (characteristic value Q_{(5.8)}) relative to the volume distribution of the droplets is between 50% and 100%, and
(c) the particle size below which 50% of the quantity of particles are found, relative to the volume distribution of the individual particles (the parameter X₅₀), is between 1 µm and 6 µm.

9. Inhalable powder according to claim 8, **characterised in that** the particles have a specific surface of between 1 m²/g and 10 m²/g.

10. Inhalable powder according to one of claims 8 or 9, **characterised in that** the acid addition salt is selected from the group consisting of 1*-*[*N²-*[3,5-dibromo-*N*-[[4-(3,4-dihydro-2(1*H*)-oxoquinazolin-3-yl)-1-piperidinyl] carbonyl]-D-tyrosyl]-L-lysyl]-4-(4-pyridinyl)-piperazine hydrochloride, sulphate, phosphate, hydrobromide, carbonate, methanesulphonate, nitrate, citrate, malate, tartrate, lactate, succinate, gluconate, acetate, formate, propionate, capronate, oxalate, maleate, fumarate, mandelate, hydroxysuccinate and *p*-toluenesulphonate,

11. Inhalable powder according to one of claims 8 or 9, **characterised in that** the acid addition salt is selected from the group consisting of 1-[*N*²-[3,5-dibromo-*N*-[[4-(3,4-dihydro-2(1*H*)-oxoquinazolin-3-yl)-1-piperidinyl]carbonyl]-D-tyrosyl]-L-lysyl]-4-(4-pyridinyl)-piperazine-hydrochloride, sulphate and hydrobromide.

12. Inhalable powder according to one of claims 8 or 9, **characterised in that** the acid addition salt is 1-[*N*²-[3,5-dibromo-*N*-[[4-(3,4-dihydro-2(1*H*)-oxoquinazolin-3-yl)-1-piperidinyl]carbonyl] -D-tyrosyl]-L-lysyl]-4-(4-pyridinyl)-piperazine hydrochloride.

13. Inhalable powder according to one of claims 8 to 12, which may be obtained by a process according to one of claims 1 to 7.

## Revendications

1. Procédé de préparation d'un inhalant en poudre, comprenant les étapes de
(a) dissolution d'un sel d'addition avec un acide de la substance active sous forme de base 1-[N²-[3,5-dibromo-N-[[4-(3,4-dihydro-2(1H)-oxoquinazolin-3-yl)-1-pipéridinyl]carbonyl]-D-tyrosyl]-L-lysyl]-4-(4-pyridinyl)-pipérazine [BIBN4096] de formule 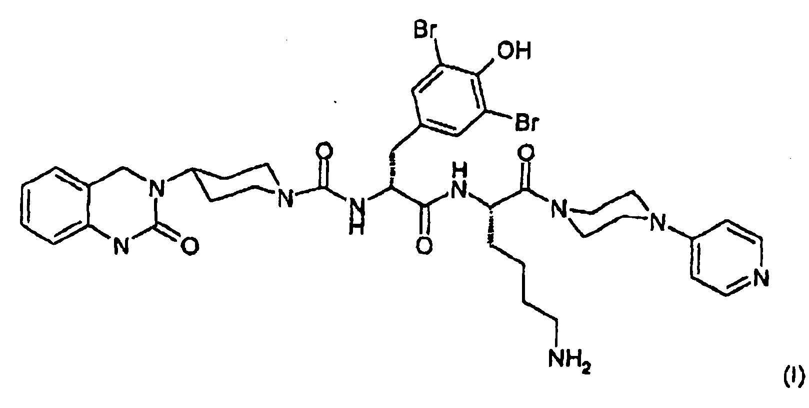 ou
dissolution de la substance active BIBN4096 dans un système aqueux, contenant un excès par rapport à la quantité de substance active à dissoudre, de préférence de 0,9 à 1,1 équivalent, d'un acide minéral ou d'un acide organique, ce qui transforme la substance active en sel correspondant,
(b) pulvérisation de la solution de substance active ainsi obtenue de manière usuelle, de manière à produire un nuage de pulvérisation, dans lequel 50 % de la quantité de particules obtenue par rapport à la répartition en volume des gouttes (valeur caractéristique X₅₀) se situe à une taille de gouttes de 1,5 à 30 µm, de préférence de 1,5 à 20 µm, de manière particulièrement préférée de 1,5 à 8 µm, et la quantité de particules, qui se situe par rapport à la répartition en volume des gouttelettes au-dessous de 5,8 µm (valeur caractéristique Q_{(5,8)}), entre 10 % et 100 %, de préférence entre 20 % et 100 %, de manière particulièrement préférée entre 50 % et 100 %,
(c) séchage du nuage de pulvérisation ainsi obtenu à l'aide d'un gaz de séchage en utilisant les paramètres suivants :
• une température d'entrée du gaz de séchage de 100°C à 350 °C, de préférence entre 120 °C et 250 °C et de manière particulièrement préférée entre 130 °C et 200 °C,
• une température de sortie du gaz de séchage de 40 °C à 120 °C,
• un courant en volume du gaz de pulvérisation de 1 Nm³/h à 15 Nm³ /h et
• un courant en volume du gaz de séchage de 15 Nm³/h à 1500 Nm³/h, de préférence de 15 Nm³/h à 150 Nm³/h, et
(d) séparation de la partie de matière solide séchée du courant de gaz de séchage de manière usuelle.

2. Procédé selon la revendication 1, **caractérisé en ce que** la solution de substance active utilisée pour le séchage par pulvérisation présente une concentration de 0,5 à 25 % en poids.

3. Procédé selon la revendication 1, **caractérisé en ce que** la solution de substance active utilisée pour le séchage par pulvérisation présente une concentration de 1 à 20 % en poids.

4. Procédé selon la revendication 1, **caractérisé en ce que** la solution de substance active utilisée pour le séchage par pulvérisation présente une concentration de 2 à 10 % en poids.

5. Procédé selon l'une des revendications 1 à 4, **caractérisé en ce que** le sel d'addition avec un acide est choisi dans le groupe constitué du chlorhydrate, sulfate, phosphate, bromhydrate, carbonate, méthanesulfonate, nitrate, citrate, maléate, tartrate, lactate, succinate, gluconate, acétate, formiate, propionate, capronatc, oxalate, maléate, fumarate, mandélate, hydroxy-succinate et p-toluènesulfonate de 1-[N²-[3,5-dibromo-N-[[4-(3,4-dihydro-2(1H)-oxoquinazolin-3-yl)-1-pipéridinyl]carbonyl]-D-tyrosyl]-L-lysyl]-4-(4-pyridinyl)-pipérazine.

6. Procédé selon l'une des revendications 1 à 4, **caractérisé en ce que** le sel d'addition avec un acide est choisi dans le groupe constitué du chlorhydrate, sulfate et bromhydrate de 1-[N²-[3,5-dibromo-N-[[4-(3,4-dihydro-2(1H)-oxoquinazolin-3-yl)-1-pipéridinyl]carbonyl]-D-tyrosyl]-L-lysyl]-4-(4-pyridinyl)-pipérazine.

7. Procédé selon l'une des revendications 1 à 4, **caractérisé en ce que** le sel d'addition avec un acide est choisi dans le groupe constitué du chlorhydrate de 1-[N²-[3,5-dibromo-N-[[4-(3,4-dihydro-2(1H)-oxoquinazolin-3-yl)-1-pipéridinyl]carbonyl]-D-tyrosyl]-L-lysyl]-4-(4-pyridinyl)-pipérazine.

8. Inhalant en poudre, constitué d'un sel d'addition avec un acide de la substance active sous forme de base 1-[N²-[3,5-dibromo-N-[[4-(3,4-dihydro-2(1H)-oxoquinazolin-3-yl)-1-pipéridinyl]carbonyl]-D-tyrosyl]-L-lysyl]-4-(4-pyridinyl)-pipérazine [BIBN4096] de formule 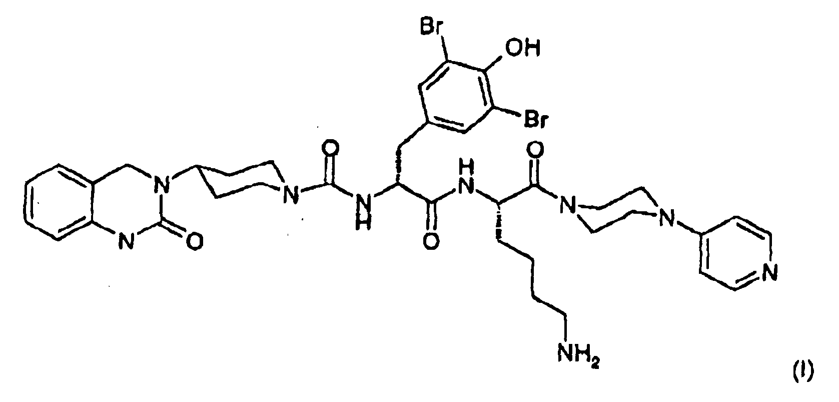 sous la forme de particules nanostructurées sphériques, **caractérisé en ce que**
(a) les particules présentent une surface spécifique entre 1 m²/g et 20 m²/g,
(b) la quantité de particules, qui se situe au-dessous de 5,8 µm (valeur caractéristique Q_{(5.8)}), par rapport à la répartition en volume des gouttelettes, est entre 50 % et 100 % et
(c) la taille de particules, au-dessous de laquelle se situe 50 % de la quantité de particules par rapport à la répartition en volume des particules individuelles est entre 1 µm et 6 µm.

9. Inhalant en poudre selon la revendication 8, **caractérisé en ce que** les particules présentent un surface spécifique entre 1 m²/g et 10 m²/g.

10. Inhalant en poudre selon l'une des revendications 8 ou 9, **caractérisé en ce que** le sel d'addition avec un acide est choisi dans le groupe constitué du chlorhydrate, sulfate, phosphate, bromhydrate, carbonate, méthanesulfonate, nitrate, citrate, maléate, tartrate, lactate, succinate, gluconate, acétate, formiate, propionate, capronate, oxalate, maléate, fumarate, mandélate, hydroxysuccinate et p-toluènesulfonate de 1-[N²-[3,5-dibromo-N-[[4-(3,4-dihydro-2(1H)-oxoquinazolin-3-yl)-1-pipéridinyl]carbonyl]-D-tyrosyl]-L-lysyl]-4-(4-pyridinyl)-pipérazine.

11. Inhalant en poudre selon l'une des revendications 8 ou 9, **caractérisé en ce que** le sel d'addition avec un acide est choisi dans le groupe constitué du chlorhydrate, sulfate et bromhydrate de 1-[N²-[3,5-dibromo-N-[[4-(3,4-dihydro-2(1H)-oxoquinazolin-3-yl)-1-pipéridinyl]carbonyl]-D-tyrosyl]-L-lysyl]-4-(4-pyridinyl)-pipérazine.

12. Inhalant en poudre selon l'une des revendications 8 ou 9, **caractérisé en ce que** le sel d'addition avec un acide est le chlorhydrate de 1-[N²-[3,5-dibromo-N-[[4-(3,4-dihydro-2(1H)-oxoquinazolin-3-yl)-1-pipéridinyl]carbonyl]-D-tyrosyl]-L-lysyl]-4-(4-pyridinyl)-pipérazine.

13. Inhalant en poudre selon l'une des revendications 8 à 12, pouvant être obtenu conformément à un procédé selon l'une des revendications 1 à 7.
